# EUROPEAN PATENT APPLICATION

(11) **EP 4 413 962 A1**
(43) Date of publication of application: **14.08.2024**
(21) Application number: 24154021.0
(22) Date of filing: 25.01.2024
(51) Int. Cl.: A61F 13/02, A61F 13/01, A61F 13/00, D04B 21/16

(54) **MEDICAL BANDAGE**

(30) Priority: 13.02.2023 CN 202320199543 U
(71) Applicant: Changzhou Beifei Non-Woven Fabric Technology Co., Ltd., Jiangsu 213000 (CN)
(72) Inventor: ZHANG, Yanbing, 213000, Jiangsu (CN)
(74) Representative: Sykora & König Patentanwälte PartG mbB

(57) **Abstract**

The invention discloses a medical bandage, and relates to the technical field of medical supplies. One specific implementation mode of the medical bandage comprises: a bandage body and an adhesive layer provided on the surface of the bandage body; the bandage body including: a non-woven fabric base and a plurality of elastic reinforcing filaments that are sewn into the non-woven fabric base and extend in the warp direction of the non-woven fabric base; wherein the non-woven fabric base is provided with a plurality of cutting positions that are arranged at equal intervals in the warp direction; each cutting position is provided with at least one linear cutting opening that extends in the weft direction of the non-woven fabric base; and each elastic reinforcing filament punctures the non-woven fabric base up and down to form continuous coils surrounding the non-woven fabric base. The implementation mode enables easily tearing apart with bare hands in the weft direction, and meanwhile has a certain strength and a certain elasticity in the warp direction.

## Description

### Technical Field

The present application relates to the technical field of medical supplies, and in particular to a medical bandage.

### Background Art

At present, medical self-adhesive non-woven bandages are made by using a polypropylene (PP) spunbond non-woven fabric as the base material, and spraying adhesives on the non-woven fabric, and the bandages basically appear in the form of rolls. When the rolled bandage is unfolded, the direction in which the bandage continuously extends is called the warp direction (that is, the length direction of the bandage), and the direction perpendicular to the warp direction is called the weft direction (that is, the width direction of the bandage). When the bandage is used, the entire roll generally will not be used up at once, and in order to facilitate the use next time, the bandage will be torn apart with bare hands, like an electrical tape, without using tools such as scissors in general cases. The production process of the spunbond non-woven fabric is that continuous spinning is performed vertically downwards, and the mesh formed below is continuously advanced in the horizontal direction and then hot-rolled and bonded, so such non-woven fabric has a continuous warp direction as the main vein direction, the fabric can generally be easily torn apart with bare hands in the warp direction, but it is extremely laborious to cut the fabric off in the weft direction. In other words, the strength of such non-woven bandage in the warp direction (that is, the strength exhibited when the bandage is stretched in the warp direction) is comparatively high (the higher the strength in the warp direction is, the harder the bandage is torn apart in the weft direction, i.e., the more difficultly the bandage is torn apart in the weft direction), and the strength in the weft direction (that is, the strength exhibited when the bandage is stretched in the weft direction) is comparatively low (the higher the strength in the weft direction is, the harder the bandage is torn apart in the warp direction, i.e., the more difficultly the bandage is torn apart in the warp direction).

The existing production process of self-adhesive non-woven bandages has been around for more than 10 years. In order to optimize the effect of tearability in the weft direction during use, many methods, e.g., changing the spinneret holes to be thinner to make the diameter of each filament finer so as to reduce the strength of the non-woven fabric in the warp direction; adding some additives during the production of the non-woven fabric base material, have been developed. These two methods will increase the production cost and the complexity of the production process without significantly reducing the difficulty of tearing the bandage apart in the weft direction.

Another method is to change the original normal spinning angle and change the direction of the nozzle from the one perpendicular to the fabric surface to a tilted direction. After such changes, the warp direction is not used as the main vein direction of the mesh, which reduces the strength of the fabric in the warp direction, and improves to some extent the tearability of the fabric in the weft direction, and the non-woven fabric made by this method is called the tearable non-woven fabric upstream and downstream of the bandage industry. However, the production of such tearable non-woven fabric belongs to a practice of producing defective products to some extent. The surface texture of the non-woven fabric is messy, and many veins tilted to the warp and weft directions, which are long or short with uneven thicknesses, may be seen. The thickness differences in both the warp direction and the weft direction of the fabric are larger than those of the ordinary non-woven fabric, and there are more lumps. Besides, such tilted spraying method wastes more raw materials than spraying at a normal angle. In addition, although the non-woven fabric made by changing the direction of the nozzle is called the tearable non-woven fabric in the industry, it is only slightly tearable as compared to the ordinary non-woven fabric, and the actual effect of tearing it apart with bare hands is also very limited and cannot meet actual requirements.

### Summary

In view of the above, embodiments of the invention provide a new type of medical bandage, which can be easily torn apart with bare hands in the weft direction, and meanwhile has a certain strength and a certain elasticity in the warp direction.

In order to achieve the aforesaid object, the invention provides a medical bandage.

The medical bandage according to an embodiment of the invention comprises: a bandage body and an adhesive layer provided on the surface of the bandage body; the bandage body including: a non-woven fabric base and a plurality of elastic reinforcing filaments that are sewn into the non-woven fabric base and extend in the warp direction of the non-woven fabric base; wherein the non-woven fabric base is provided with a plurality of cutting positions that are arranged at equal intervals in the warp direction; each cutting position is provided with at least one linear cutting opening that extends in the weft direction of the non-woven fabric base; and each elastic reinforcing filament punctures the non-woven fabric base up and down to form continuous coils surrounding the non-woven fabric base.

Optionally, the lengths of the cutting openings in the plurality of cutting positions are equal.

Optionally, the distances between arbitrary two adjacent cutting openings in the weft direction in the same cutting position are equal.

Optionally, the distances between adjacent cutting openings in the weft direction in the plurality of cutting positions are equal.

Optionally, for an arbitrary cutting opening in an arbitrary cutting position, there is only one cutting opening in each of the other cutting positions that is flush with the arbitrary cutting opening in the warp direction of the non-woven fabric base.

Optionally, the plurality of elastic reinforcing filaments are arranged at equal intervals in the weft direction of the non-woven fabric base.

Optionally, when no external force is applied, the non-woven fabric base is in a shrunk and wrinkled state in the warp direction under the action of the elastic reinforcing filaments.

Optionally, when a stretching force in the warp direction is applied, the length of the bandage body in the warp direction is larger than the initial length, and the non-woven fabric base is in a flat state.

Optionally, the number of the elastic reinforcing filaments included in the medical bandage with a width of one inch in the weft direction is 7, 9 or 12.

Optionally, the number of the adhesive layers is 2, and the two adhesive layers are provided on the two surfaces of the bandage body, respectively.

According to the technical solution of the invention, the aforesaid embodiment of the invention has the following advantages or beneficial effects:

The medical bandage according to the embodiment of the invention comprises a bandage body and an adhesive layer, the adhesive layer is provided on the surface of the bandage body, the bandage body includes a non-woven fabric base, the non-woven fabric base is provided with a plurality of cutting positions that are arranged at equal intervals in the warp direction, and each cutting position is provided with at least one linear cutting opening that extends in the weft direction of the non-woven fabric base. Based on the above cutting position and cutting opening, the effect of easily tearing the bandage apart with bare hands in the weft direction can be achieved without significantly increasing the production cost and the production complexity; by adjusting parameters such as the length of the cutting opening, the distance between the cutting openings in the weft direction in the same cutting position, and the arrangement interval between the cutting positions in the warp direction, the difficulty of tearing the bandage apart with bare hands and the selection of the position in which the bandage is torn apart in the warp direction can also be flexibly adjusted. More preferably, the above bandage body further includes a plurality of elastic reinforcing filaments, which extend in the warp direction of the non-woven fabric base, and puncture the non-woven fabric base up and down to form continuous coils surrounding the non-woven fabric base so as to be sewn into the non-woven fabric base, and the elastic reinforcing filament with the above structure can enhance the strength of the bandage in the warp direction that was damaged by the above cutting openings, which prevents the weakening of the strength in the warp direction from causing inconvenience to the normal use of the bandage without influencing the original effect of tearing the bandage apart with bare hands in the weft direction. The above elastic reinforcing filaments also make the bandage have a certain elasticity, which facilitates bandaging and fixation. In optional production scenarios, the above elastic reinforcing filaments may be pre-stretched when being sewn into the non-woven base, so that the produced non-woven fabric base is in a shrunk and wrinkled state in the warp direction in the natural state (that is, no external force is applied). The length of such bandage can reach several times the initial length after being fully stretched in the warp direction, and the above shrunk and wrinkled state can improve the effect of breathability of the bandage, and make the bandage suitable for various medical rescue situations.

Further effects of the aforesaid non-conventional optional manners will be described below in combination with specific implementation modes.

### Brief Description of the Drawings

Figures are used to better understand the invention, and do not form improper limitations of the invention. Where:
FIG 1 is a schematic diagram of a shape of a non-woven fabric base and an arrangement of cutting openings in an embodiment of the invention;
FIG 2 is a schematic diagram of a shape and a structure of a bandage body in an embodiment of the invention; and
FIG 3 is a schematic diagram of an elastic reinforcing filament surrounding the non-woven fabric base in an embodiment of the invention.

### Explanations of reference signs:

10: non-woven fabric base; 11: cutting opening; 20: blade; 30: elastic reinforcing filament; 31: coil; 40: bandage body.

### Detailed Description

The exemplary embodiments of the invention, including various details of the embodiments of the invention, are described below in combination with the figures to facilitate understanding, and shall be considered to be exemplary ones only. Thus, those skilled in the art should recognize that various changes and modifications may be made to the embodiments described herein without departing from the scope and spirit of the invention. Similarly, for clarity and conciseness, descriptions of well-known functions and structures are omitted in the descriptions below.

It should be mentioned that "arrangement", "installation" and "connection" in the descriptions below may express a fixed fit between two objects, or a non-fixed fit (such as a sliding connection); they may express a detachable fit (such as a bolt or nut connection) or a non-detachable fit (such as welding); they may express a direct fit, or an indirect fit using a third object as a medium. In addition, where there is no conflict, the embodiments of the invention and the technical features in the embodiments may be combined with each.

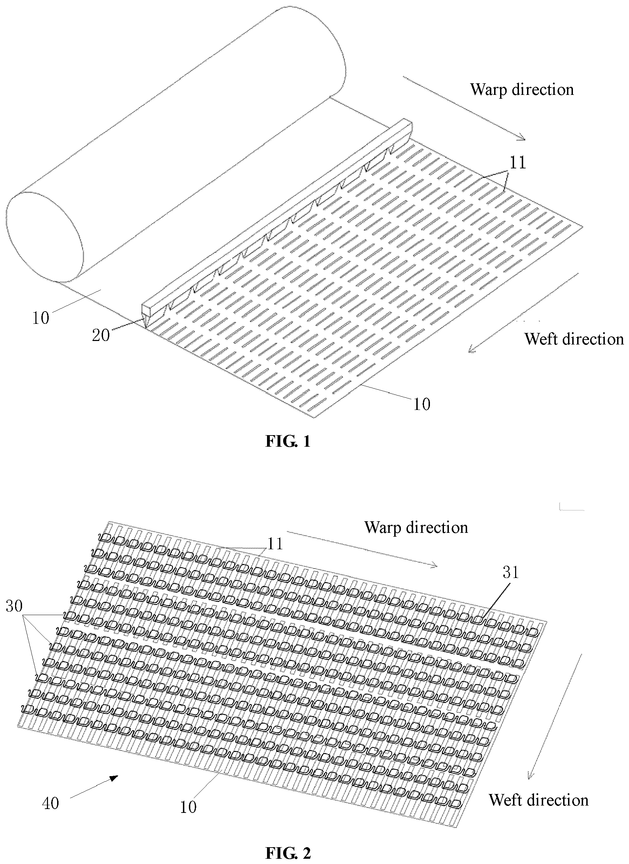

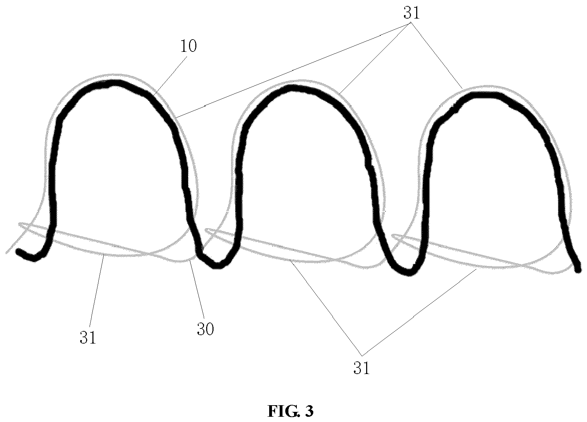

FIG. 1 is a schematic diagram of a shape of a non-woven fabric base and an arrangement of cutting openings in an embodiment of the invention, FIG. 2 is a schematic diagram of a shape and a structure of a bandage body in an embodiment of the invention, and FIG. 3 is a schematic diagram of an elastic reinforcing filament surrounding the non-woven fabric base in an embodiment of the invention. As shown in FIG. 1 to FIG. 3, the medical bandage according to the embodiment of the invention comprises a bandage body 40, the bandage body 40 including a non-woven fabric base 10 and a plurality of elastic reinforcing filaments 30 pre-sewn into the non-woven fabric base 10.

In the embodiment of the invention, the above non-woven fabric base 10 may be a non-woven fabric using various materials and production processes, and is preferably a polypropylene spunbond non-woven fabric; the above elastic reinforcing filaments 30 may be various filaments with a certain elasticity and a certain stretching strength, and are preferably spandex filaments.

In order to make the medical bandage be easily torn apart with bare hands in the weft direction, a plurality of cutting positions may be provided on the above non-woven fabric base 10. These cutting positions are linear and arranged at equal intervals in the warp direction. Each cutting position includes at least one linear cutting opening 11 and a non-woven fabric area adjacent to the cutting opening 11, and each of the above cutting openings 11 extends in the weft direction of the non-woven fabric base 10. It may be understood that when the medical bandage is torn apart with bare hands along the cutting positions, the existence of the above cutting openings 11 can reduce the amount of force required for tearing the bandage apart. In practical applications, the interval at which the above cutting positions are arranged in the warp direction may be adjusted. If the arrangement interval in the warp direction is comparatively large, the bandage may be torn apart in a limited number of cutting positions; if the arrangement interval in the warp direction is comparatively small, the effect of tearing the bandage apart anywhere can be achieved. For example, the above cutting openings may be formed by physical cutting with blades, or may be formed by using ultrasonic waves, laser engraving devices, or other suitable cutting manners.

As a better technical solution, the above cutting position and cutting opening 11 can also be subjected to the following optimization. First, the lengths of the cutting openings 11 in the above plurality of cutting positions may be designed to be equal, which is beneficial to the medical bandage having similar amounts of force required for tearing the bandage apart in each cutting position; and the lengths of the above cutting openings 11 may also be flexibly adjusted to correspond to different amounts of force required for tearing the bandage apart. Second, on this basis, the distances between arbitrary two adjacent cutting openings 11 in the weft direction in the same cutting position may be designed to be equal, and the distances between adjacent cutting openings 11 in the weft direction in each cutting position may be further designed to be equal, which can also make the tearing attributes of medical bandages be more standardized; and it is possible to change the amount of force required for tearing the bandage apart by adjusting the above distances in the weft direction. Third, the layout of the cutting openings 11 may be designed as follows: for an arbitrary cutting opening in an arbitrary cutting position, there is only one cutting opening in each of the other cutting positions that is flush with the arbitrary cutting opening in the warp direction of the non-woven fabric base. The content "flush with ... in the warp direction" refers to that in a plane rectangular coordinate system with the warp direction as the horizontal axis and the weft direction as the vertical axis, the starting points of the two cutting openings 11 have the same ordinate, and the end points thereof also have the same ordinate. It may be understood that in such layout, the distribution positions of the cutting openings 11 in each cutting position are exactly the same, and such layout facilitates large-scale production of medical bandages and can also ensure consistency of product attributes and qualities. A non-woven fabric base with the above three characteristics is as shown in FIG. 1 and FIG. 2.

In the embodiment of the invention, the above interval at which the cutting positions are arranged in the warp direction may be further adjusted for further optimization for the reason that as mentioned above, the comparatively small arrangement interval in the warp direction can achieve the effect of tearing the bandage apart anywhere, but if the arrangement interval is too small, it will easily lead to misalignment and unevenness of tearing lines, which influences the use effect of the medical bandage, so the minimum value of the arrangement interval in the warp direction may be set in accordance with the theoretical calculation and the actual use. For example, this minimum value may be the length of the cutting opening, the distance between the cutting openings in the weft direction, the sum of the length of the cutting opening and the distance between the cutting openings in the weft direction, or the like.

It may be understood that the above design of the cutting position and the cutting opening 11 achieve the convenience of tearing the bandage apart in the weft direction by reducing the strength of the non-woven fabric in the warp direction, but the reduction of the strength in the warp direction may bring inconvenience to the use of the medical bandage. For example, when performing bandaging using a medical bandage, the medical bandage breaks in a cutting position where it is not expected to break, so it is necessary to enhance the strength of the medical bandage in the warp direction without influencing the above effect of tearing the bandage apart in the weft direction. In the technical solution of the embodiment of the invention, a plurality of elastic reinforcing filaments 30 are sewn into the above non-woven fabric base 10, the above elastic reinforcing filaments 30 extend in the warp direction of the non-woven fabric base 10, each elastic reinforcing filament 30 punctures the non-woven fabric base 10 up and down to form continuous coils 31 surrounding the non-woven fabric base 10 so as to be sewn into the non-woven fabric base 10, and the non-woven fabric base 10 and the elastic reinforcing filaments 30 sewn thereinto form the bandage body 40.

In practical applications, the above elastic reinforcing filaments 30 may be spandex filaments with an excellent elasticity, and the elasticity ratio thereof can reach at least 1:5 (that is, the length of the stretched filament is at least 5 times the initial length). As shown in FIG 2 and FIG 3, a continuous elastic reinforcing filament 30 punctures the non-woven fabric base 10 up and down to form a plurality of continuous coils 31 surrounding the non-woven fabric base 10 (the respective coils 31 are connected by the same elastic reinforcing filament 30, and thus are called continuous coils) to thereby ensure the connection strength of the non-woven fabric and the elastic reinforcement filament 30, enhance the strength of the non-woven fabric base 10 in the warp direction to avoid inconvenience in use that may be caused by the previous reduction of the strength in the warp direction, and the elastic reinforcing filament 30 with the above structure will not influence the effect of tearing the non-woven fabric base 10 apart in the weft direction.

As a preferred solution, the above plurality of elastic reinforcing filaments 30 may be designed to be arranged at equal intervals in the weft direction of the non-woven fabric base 10. For example, the particular specification may be as follows: each inch of medical bandage includes 7, 9 or 12 elastic reinforcing filaments, that is, the number of the elastic reinforcing filaments included in the medical bandage with a width of one inch in the weft direction is 7, 9 or 12.

In actual production processes, the elastic reinforcing filaments 30 may be pre-stretched and then sewn into the non-woven fabric base 10, and after this step, when no external force is applied, the non-woven fabric base 10 that is originally flat is in a shrunk and wrinkled state in the warp direction under the action of the elastic reinforcing filaments 30 (at this time, the elastic reinforcing filaments return to the natural state). Apparently, the bandage body 40 in this state has a certain elasticity, and when a stretching force in the warp direction is applied, the length of the bandage body 40 in the warp direction will be larger than the initial length, and at this time, the non-woven fabric base 10 is in a flat state relative to the above shrunk and wrinkled state. Such elasticity facilitates the bandaging stability of the medical bandage, the bandaged area can also carry out appropriate activities, and meanwhile the above shrunk and wrinkled state will improve the effect of breathability of the medical bandage.

Preferably, the medical bandage according to the embodiment of the invention also comprises an adhesive layer, and the number of the adhesive layers may be one or two. In a case of one adhesive layer, the adhesive layer is adhered to one surface of the bandage body 40; more preferably, in a case of two adhesive layers (explanations below are mainly given in this case), the adhesive layers are adhered to both surfaces of the bandage body 40 to thereby form a self-adhesive elastic bandage. The above adhesive layer may be formed by using natural rubber as the main raw material, the self-adhesive strength of the natural rubber is very high, after the adhesive layers are coated on both surfaces of the bandage body, the individual two surfaces will not stick to skin, clothes, and so on, but after the bandage is wound in an overlapping manner, the adhesive layers on the upper and lower surfaces can stick together when they come into contact with each other, to thereby fix the bandage that has been wound in an overlapping manner.

A specific embodiment of the invention and a corresponding production process are introduced below.

A polypropylene (PP) spunbond non-woven fabric is the main base material of the self-adhesive elastic bandage. Such non-woven fabric is shaped by laying a mesh of continuous filaments in the warp direction, and this process characteristic makes it difficult to cut the bandage off in the weft direction without using a sharp tool. However, during the actual use of the bandage, the bandage must often be required to be cut off in the weft direction, and for the convenience of use, if it is desired to easily tear the bandage apart with bare hands, the strength of the bandage in the warp direction appears to be more than needed. The principle of the embodiment is just to damage the strength of the non-woven fabric base in the warp direction in advance, and then enable the non-woven fabric base to obtain a considerable elasticity in the warp direction by a filament adding step (filament adding refers to sewing elastic reinforcement filaments in), so that the non-woven fabric base has an enhanced strength in the warp direction while becoming an elastic bandage.

The first main step is the cutting step. The non-woven fabric base is cut with disconnected blades in the weft direction in advance, so that the non-woven fabric base may be easily torn apart with bare hands along the disconnected cutting openings in the weft direction. There are two things that can be adjusted in this step, one is the length of the cutting opening and the distance between the cutting openings in the weft direction, the adjustment of which is used to adjust the amount of force required for tearing the bandage apart with bare hands, for example, the cutting opening of the non-woven fabric that is comparatively thick is required to be longer, and the distance between the cutting openings in the weft direction should also be reduced correspondingly to ensure that the bandage can be easily torn apart with bare hands; the other is the distance between the cutting openings in the warp direction, and the smaller the distance is, the more easily the effect of tearing the bandage apart anywhere may be achieved. The above cutting step should be completed before the filament adding step.

The second main step is the filament adding step. A large stitching machine is used to sew elastic reinforcing filaments (such as spandex filaments) into a cut non-woven fabric base. The polypropylene spunbond non-woven fabric itself is inelastic. This step is to sew the elastic reinforcement filaments into the non-woven fabric in the form of continuous coils in the warp direction, and the particular specification may be as follows: each inch of medical bandage includes 7, 9 or 12 elastic reinforcing filaments, so that the non-woven fabric base obtains an elasticity in the warp direction. The elasticity ratio of the spandex filament itself before and after the stretching can reach at least 1:5, the spandex filament is pre-stretched in the filament adding step, after the filament adding step, the non-woven fabric that is originally flat will be in a shrunk and wrinkled state in the warp direction, and the ratio of the bandage body, which has been subjected to the filament adding step, before and after the sufficient stretching can reach at least 1:2. Another important change in the non-woven fabric after the filament adding step is that the strength in the warp direction is basically determined by the strength of the elastic reinforcing filament, and the strength of non-woven fabric base in the warp direction that was damaged in the first cutting step is enhanced.

The non-woven fabric that has been subjected to the filament adding step looks like a shrunk, wrinkled and fluffy one as seen from the surface in the natural state, and if a force is applied on the left and right sides in the warp direction, the wrinkled fabric wrapped by the elastic reinforcing filaments may be stretched, the wrinkles on the fabric surface will also become gentle, and the original shrunk and wrinkled state can be returned to after the release. During the actual use, the bandage can still have a sufficient elastic effect after being wrapped, and the bandaged area can also carry out appropriate activities, which is very suitable for various medical rescue situations, and can improve the key effect of breathability depending on this shrunk, wrinkled and fluffy state.

The third step is the adhering step. A special adhesive made by using natural rubber as the main raw material is coated on both surfaces of the bandage body, the self-adhesive strength of the natural rubber is very high, after the adhesives are coated on both surfaces of the bandage body, the individual two surfaces will not stick to skin, clothes, and so on, but after the bandage is wound in an overlapping manner, the adhesives on the upper and lower surfaces can stick together when they come into contact with each other, to thereby fix the bandage that has been wound in an overlapping manner.

Those skilled in the art should know that the aforesaid embodiments described in the Description all belong to preferred embodiments, and the components involved are not necessarily indispensable to the implementation of the invention.

According to the technical solution of the embodiment of the invention, the effect of tearing the bandage apart with bare hands in the weft direction can be significantly improved by arranging the cutting position and the cutting opening, the difficulty of tearing the bandage apart in the weft direction may be adjusted by adjusting the length of the cutting opening, so that the amount of force required for tearing the bandage apart with bare hands in the warp direction is controllable and adjustable; and the interval between the cutting positions in the warp direction may also be adjusted, so that when the bandage is required to be torn apart with bare hands during the actual use, the effect of freely selecting the length of use can be achieved. The above technical effects cannot be achieved by the prior art, and meanwhile the above solution will not significantly increase the production cost and the production complexity. In addition, dense and parallel elastic reinforcing filaments are sewn into the non-woven fabric base in the warp direction thereof, which turns an ordinary non-woven fabric into an elastic non-woven fabric, enhances the strength of non-woven fabric base in the warp direction that was damaged, and provides a better effect of breathability by fluffy wrinkles produced by natural shrinkage.

The above specific implementation modes do not constitute limitations on the scope of protection of the invention. It should be understood by those skilled in the art that various modifications, combinations, sub-combinations and substitutions may occur depending on design requirements and other factors. Any modification, equivalent replacement, improvement, and so on made within the spirit and principle of the invention should be included in the scope of protection of the invention.

## Claims

1. A medical bandage, **characterized by** comprising: a bandage body and an adhesive layer provided on the surface of the bandage body;
the bandage body including: a non-woven fabric base and a plurality of elastic reinforcing filaments that are sewn into the non-woven fabric base and extend in the warp direction of the non-woven fabric base; wherein
the non-woven fabric base is provided with a plurality of cutting positions that are arranged at equal intervals in the warp direction;
each cutting position is provided with at least one linear cutting opening that extends in the weft direction of the non-woven fabric base; and
each elastic reinforcing filament punctures the non-woven fabric base up and down to form continuous coils surrounding the non-woven fabric base.

2. The medical bandage according to claim 1, **characterized in that** the lengths of the cutting openings in the plurality of cutting positions are equal.

3. The medical bandage according to claim 2, **characterized in that** the distances between arbitrary two adjacent cutting openings in the weft direction in the same cutting position are equal.

4. The medical bandage according to claim 3, **characterized in that** the distances between adjacent cutting openings in the weft direction in the plurality of cutting positions are equal.

5. The medical bandage according to claim 4, **characterized in that** for an arbitrary cutting opening in an arbitrary cutting position, there is only one cutting opening in each of the other cutting positions that is flush with the arbitrary cutting opening in the warp direction of the non-woven fabric base.

6. The medical bandage according to claim 1, **characterized in that** the plurality of elastic reinforcing filaments are arranged at equal intervals in the weft direction of the non-woven fabric base.

7. The medical bandage according to claim 1, **characterized in that** when no external force is applied, the non-woven fabric base is in a shrunk and wrinkled state in the warp direction under the action of the elastic reinforcing filaments.

8. The medical bandage according to claim 7, **characterized in that** when a stretching force in the warp direction is applied, the length of the bandage body in the warp direction is larger than the initial length, and the non-woven fabric base is in a flat state.

9. The medical bandage according to claim 6, **characterized in that** the number of the elastic reinforcing filaments included in the medical bandage with a width of one inch in the weft direction is 7, 9 or 12.

10. The medical bandage according to claim 1, **characterized in that** the number of the adhesive layers is 2, and the two adhesive layers are provided on the two surfaces of the bandage body, respectively.
